# EUROPÄISCHE PATENTANMELDUNG

(11) **EP 2 386 356 A1**
(43) Veröffentlichungstag der Anmeldung: **16.11.2011**
(21) Anmeldenummer: 10005000.4
(22) Anmeldetag: 12.05.2010
(51) Int. Cl.: B01L 3/00, B01L 3/02, G01N 33/28

(54) **Probeentnahme-Behälter für eine Flüssigkeit mit Behälter-Tag**

(71) Anmelder: ABB Technology AG, 8050 Zürich (CH)
(72) Erfinder: Fantana, Nicolaie L., 69124 Heidelberg (DE); Radigk, Cornelia, 06124 Halle (DE); Kouzmine, Oleg, 40217 Düsseldorf (DE); Werle, Peter, 29664 Walsrode (DE)
(74) Vertreter: Partner, Lothar

(57) **Zusammenfassung**

Es wird ein Probeentnahme-Behälter (24, 27) für eine Flüssigkeit mit Behälter-Tag (22) und zugeordneter, lösbarer Sensoreinheit (1) vorgeschlagen,
● wobei der Behälter-Tag (22) fest am Probeentnahme-Behälter (24, 27) angebracht ist und einen via Funk auslesbaren Lese-Speicherbereich aufweist, welcher eine eindeutige Identifikation des Probeentnahme-Behälters (24, 27) ermöglicht,
● wobei die zugeordnete, lösbare Sensoreinheit (1) während der Probeentnahme lösbar am Probeentnahme-Behälter (24, 27) befestigt ist und eine Elektronikgruppe (19) inklusive Prozesseinheit (3), elektronischer Speicherschaltung (5) und Energieversorgung (15), eine via Funk zu betreibende Kommunikationsgruppe (18) sowie eine Sensorgruppe (20) aufweist, welche Vorort während der Entnahme der Flüssigkeit aus einer Leistungs-Einrichtung (33) "vorläufige Probe-Sensorik-Daten" ermittelt,
● wobei der Behälter-Tag (22) des Weiteren einen via Funk beschreibbaren/auslesbaren Schreib/Lese-Speicherbereich aufweist, welcher ein Abspeichern der von der lösbaren Sensoreinheit (1) erfassten "vorläufigen Probe-Sensorik-Daten" sowie der diese Probeentnahme betreffenden "administrativen Daten" ermöglicht.

## Beschreibung

Die Erfindung betrifft einen Probeentnahme-Behälter für eine Flüssigkeit mit Behälter-Tag.

Leistungs-Einrichtungen der Energieerzeugung und industrielle Leistungs-Einrichtungen weisen vielfach Kühlflüssigkeiten oder Arbeitsflüssigkeiten, wie Öl auf. Ein Beispiel hierfür ist ein mit Öl gefüllter Leistungstransformator. Um den ordnungsgemäßen Betrieb der Leistungs-Einrichtung dauerhaft sicherzustellen, ist es erforderlich, der Flüssigkeit in bestimmten zeitlichen Abständen eine Probe zu entnehmen. Der mit der Probe gefüllte Probeentnahme-Behälter wird danach in ein Labor transportiert, wo eine Analyse der Probe erfolgen kann. Als Probeentnahme-Behälter können einerseits Flaschen aus Aluminium, Stahl, Glas oder Kunststoff verwendet werden, andererseits auch Spritzen oder zweiseitig offene Behälter.

Um eine genaue Zuordnung zwischen der Leistungs-Einrichtung einerseits und der entnommenen Probe anderseits festzuhalten, ist es üblich, die relevanten Daten, wie Eigentümer und kennzeichnende Daten der Leistungs-Einrichtung, Entnahmeort und gewünschte Analysenwerte auf einem am Probeentnahme-Behälter befestigten Aufkleber oder einer Plakette niederzuschreiben. Dabei besteht das Risiko eines Verlustes dieser Plakette oder einer Beschädigung dieses Aufklebers. Des Weiteren ist es allgemein bekannt, einen Behälter mit einem Behälter-Tag zu versehen, welcher einen Lese-Speicherbereich aufweist, via Funk auslesbar ist und eine eindeutige Identifikation des Behälters ermöglicht.

Der Erfindung liegt die Aufgabe zugrunde, einen optimierten Probeentnahme-Behälter für eine Flüssigkeit mit Behälter-Tag anzugeben.

Diese Aufgabe wird erfindungsgemäß gelöst durch einen Probeentnahme-Behälter für eine Flüssigkeit mit Behälter-Tag und zugeordneter, lösbarer Sensoreinheit,
● wobei der Behälter-Tag fest am Probeentnahme-Behälter angebracht ist und einen via Funk auslesbaren Lese-Speicherbereich-aufweist, welcher eine eindeutige Identifikation des Probeentnahme-Behälters ermöglicht,
● wobei die zugeordnete, lösbare Sensoreinheit während der Probeentnahme lösbar am Probeentnahme-Behälter befestigt ist und eine Elektronikgruppe inklusive Prozesseinheit, elektronischer Speicherschaltung und Energieversorgung, eine via Funk zu betreibende Kommunikationsgruppe sowie eine Sensorgruppe aufweist, welche Vorort während der Entnahme der Flüssigkeit aus einer Leistungs-Einrichtung "vorläufige Probe-Sensorik-Daten" ermittelt,
● wobei der Behälter-Tag des Weiteren einen via Funk beschreibbaren/auslesbaren Schreib/Lese-Speicherbereich aufweist, welcher ein Abspeichern der von der lösbaren Sensoreinheit erfassten "vorläufigen Probe-Sensorik-Daten" sowie der diese Probeentnahme betreffenden "administrativen Daten" ermöglicht.

Die mit der Erfindung erzielbaren Vorteile bestehen insbesondere darin, dass einerseits in kostengünstiger Art und Weise Vorort wertvolle "vorläufige Probe-Sensorik-Daten" erfasst und festgehalten werden können, welche die laborseitige Analyse der Probe ergänzen, und dass andererseits eine eindeutige Identifikation der entnommenen Probe sichergestellt ist. Da die relativ kostenintensive Sensoreinheit nicht zusammen mit dem Probeentnahme-Behälter zum Labor transportiert werden muss, sondern vielmehr für weitere nachfolgende Probeentnahmen zur Verfügung steht, ergeben sich Kostenvorteile.

Zweckmäßige Ausgestaltungen der Erfindung sind in den Unteransprüchen gekennzeichnet.

Die Erfindung wird nachstehend an Hand der in der Zeichnung dargestellten Ausführungsbeispiele erläutert. Es zeigen:
- Fig. 1: eine erste Ausführungsform mit einem Probeentnahme-Behälter für eine Flüssigkeit mit Behälter-Tag und zugeordneter, lösbarer Sensoreinheit,
- Fig. 2: eine Sensoreinheit im Detail,
- Fig. 3: eine zweite Ausführungsform mit einem Probeentnahme-Behälter für eine Flüssigkeit in Form einer Spritze,
- Fig. 4: eine dritte Ausführungsform mit einem Probeentnahme-Behälter für eine Flüssigkeit mit zugeordneter, lösbarer Durchflusseinheit.
- Fig. 5a - 5f: unterschiedliche Ausführungsvarianten von ProbeentnahmeBehältern.

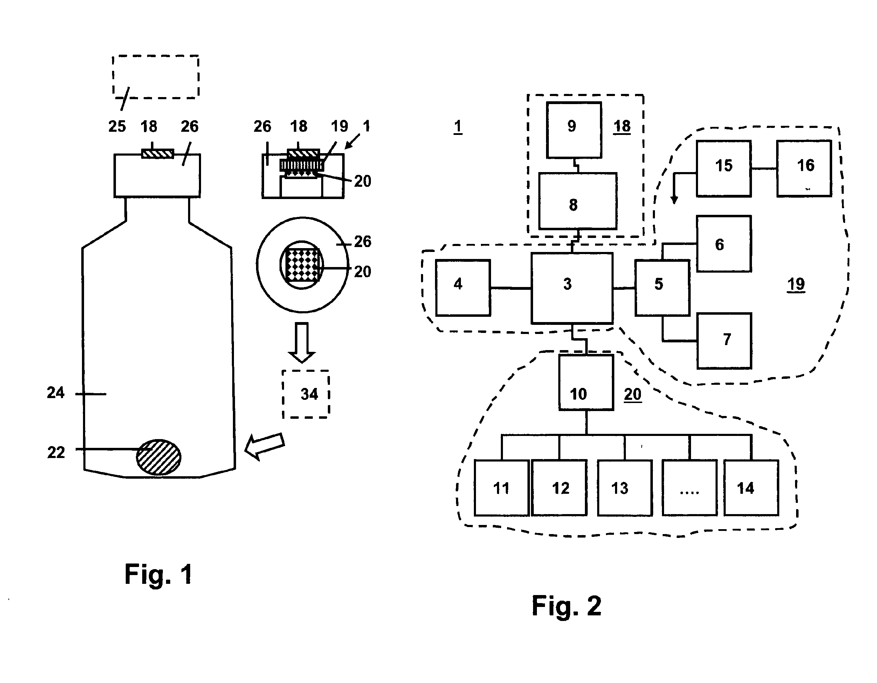

In Fig. 1 ist eine erste Ausführungsform mit einem Probeentnahme-Behälter für eine Flüssigkeit mit Behälter-Tag und zugeordneter, lösbarer Sensoreinheit dargestellt. Es ist ein z. B. aus Aluminium bestehender Probeentnahme-Behälter 24 für die Aufnahme einer Flüssigkeit, insbesondere Öl, z. B. Transformatorenöl, nachfolgend auch als Probe bezeichnet, zu erkennen, welcher mittels einer Kappe 26 verschlossen ist und an dessen Außenwand ein Behälter-Tag 22, vorzugsweise RFID-Tag (vorzugsweise vom passiven Typ) permanent angebracht, z. B. angeklebt ist. Die Kappe 26 ist mit einer Sensoreinheit 1 versehen, deren Kommunikationsgruppe 18 die Oberseite der Kappe 26 überragt.

Der Behälter-Tag 22 weist einen Lese-Speicherbereich (read-only) für das Abspeichern einer eindeutigen Identifikation auf. Der Behälter-Tag 22 weist des Weiteren einen Schreib/Lese-Speicherbereich auf, welcher zum Abspeichern folgender die Probeentnahme betreffender "administrativer Daten" geeignet ist:
● Aufstellungsort und Eigentümer der Leistungs-Einrichtung,
● kennzeichnende Daten der Leistungs-Einrichtung,
● die Leistungs-Einrichtung betreffende "historische Daten",
● Flüssigkeitstyp, z. B. Öltyp,
● genauer Entnahmeort der Probe, z. B. "im Bodenbereich", "im oberen Bereich", "im mittleren Bereich",
● Datum und Uhrzeit dieser Probeentnahme,
● Temperatur der Probe während der Entnahme,
● Name der Service-Person, welche die Probe entnommen hat,
● wichtige (kritische) Beobachtungen der Service-Person während der Entnahme der Probe,
● die vom Labor zu ermittelnden gewünschten Analysenwerte, z. B. in der Flüssigkeit gelöste Gase, durchzuführende chemische Tests, Furane, usw.

Darüber hinaus dient der Schreib/Lese-Speicherbereich des Behälter-Tags 22 zusätzlich für das Speichern von mittels der nachfolgend näher erläuterten Sensoreinheit 1 ermittelten gewünschten Sensordaten. In einem solchen Fall enthalten die Speichermittel des Behälter-Tags 22 alle Vorort während der Entnahme ermittelten, die Probe betreffenden und für das Labor wichtigen "vorläufigen Probe-Sensorik-Daten".

Damit wird durch den Behälter-Tag 22 eine eindeutige, nicht änderbare und verwechslungssichere Bezeichnung zur eindeutigen Identifizierung der Probe sichergestellt. Des Weiteren besitzt der Behälter-Tag 22 vorzugsweise einen Temperatursensor zur Ermittlung der während der Erfassung der gewünschten Probe herrschenden Temperatur (Behältertemperatur).

Bei Verwendung eines passiven RFID-Tags als Behälter-Tag 22 entspricht quasi die Behältertemperatur der augenblicklichen Temperatur zum Ablesezeitpunkt der entnommenen Flüssigkeit und wird nach dem Ablesen abgespeichert.

Bei Verwendung eines aktiven RFID-Tags als Behälter-Tag 22 können die Sensordaten in Form einer Temperaturkurve (Abhängigkeit Temperatur/Zeit) kontinuierlich während des gesamten Entnahme-Vorganges detektiert und abgespeichert werden. Es ist dann einfach möglich, eine typische Temperatur der Probe aus der Temperaturkurve abzuleiten. Danach erfolgt das Abspeichern der abgeleiteten Temperatur.

Anstelle eines RFID-Tags kann z. B. auch ein NFC-Tag (Near Field Communication) als Behälter-Tag 22 eingesetzt werden.

Fig. 1 zeigt in ihrem rechten Bereich einen seitlichen Schnitt durch eine Kappe und eine Sicht auf die Unterseite einer Kappe. Im seitlichen Schnitt durch die Kappe ist dargestellt, dass die Sensoreinheit 1 außer der Kommunikationsgruppe 18 eine Elektronikgruppe 19 inklusive Energieversorgung und eine Sensorgruppe 20 umfasst, welche unmittelbar mit dem Innenraum des auch vereinfachend als "Behälter" bezeichneten Probeentnahme-Behälters 24 in Kontakt steht. Auch in der Sicht auf die Unterseite der Kappe 26 ist die Sensorgruppe 20 gut zu erkennen. Es ist leicht zu erkennen, dass die Sensorgruppe 20 in Kontakt mit der entnommenen und im Innenraum des Behälters 24 befindlichen Probe tritt, sobald der Probeentnahme-Behälter 24 "auf den Kopf" gestellt wird.

Fig. 1 zeigt des Weiteren eine bevorzugte Möglichkeit, die vom Sensor 1 erfassten Sensordaten via Kommunikationsgruppe 18 und via Funk an ein externes Lesegerät 34 zu übertragen und dort abzuspeichern. Vom Lesegerät 34 können die Sensordaten danach via Funk an den Behälter-Tag 22 übertragen und dort abgespeichert werden. Die Kappe 26 kann vorteilhaft durch eine einfache, kostengünstige Kappe 25 ohne Sensoreinheit 1 ersetzt werden, d. h. der Transport des Behälters 24 zum Labor kann wahlweise mit Kappe 25 (was vorzuziehen ist) oder mit Kappe 26 erfolgen, wobei
● bei Transport mit der kostengünstigen Kappe 25 eine Übertragung der Daten an den Behälter-Tag 22 zwingend erforderlich ist,
● bei Transport mit der relativ teuren Kappe 26 eine Übertragung der Daten an den Behälter-Tag 22 nicht erforderlich ist.

In Fig. 2 ist eine Sensoreinheit im Detail dargestellt. Die Sensoreinheit 1 umfasst:
● die Kommunikationsgruppe 18, bestehend aus einem elektronischen RFID-Schaltkreis 8 und einer RFID-Antenne 9,
● die Elektronikgruppe 19 inklusive Energieversorgung, bestehend aus einer Prozesseinheit 3 (Mikroprozessor oder Mikrocontroller oder FPGA (Field Programmable Array)), welcher mit dem elektronischen RFID-Schaltkreis 8, mit einem digitalen Signalprozessor 4 (DSP) und mit einer elektronischen Speicherschaltung 5 verbunden ist, welche z. B. einen ersten Speicherbereich 6 und einen zweiten Speicherbereich 7 umfasst, wobei die vorstehend angesprochene Energieversorgung z. B. aus einer Batterie / einem Akkumulator 15 sowie einer induktiven Aufladeschaltung 16 für die Aufladung des Akkumulators 16 aus einem Versorgungsnetz (Wechselstromnetz) besteht,
● die Sensorgruppe 20, bestehend aus einem mit der Prozesseinheit 3 verbundenen elektronischen Sensorschaltkreis 10, an welchen ein erster Sensor 11, ein zweiter Sensor 12, ein dritter Sensor 13 ... sowie ein n-ter Sensor 14 angeschlossen sind.

Anstelle eines RFID-Schaltkreises kann z. B. auch ein NFC-Schaltkreis (Near Field Communication) mit entsprechender Antenne eingesetzt werden.

Als Sensoren 11 - 14 für die Detektion von "vorläufigen Probe-Sensorik-Daten" können insbesondere z. B. eingesetzt werden:
● Sensor zur Ermittlung von gelöstem Wasserstoff,
● Sensor zur Ermittlung von enthaltenem Azetylen,
● Sensor zur Ermittlung von enthaltenem Ethin,
● Sensor zur Ermittlung von enthaltenem Wasser,
● Sensor zur Ermittlung von enthaltenem Kohlenmonoxyd,
● Sensor zur Ermittlung von enthaltenem Kohlendioxyd,
● Sensor zur Ermittlung von enthaltenem Sauerstoff,
● Sensor zur Ermittlung von enthaltenem Stickstoff,
● Sensor zur Ermittlung der Leitfähigkeit,
● Sensor zur Ermittlung des Säuregehalts,
● Temperatursensor.

Im ersten Speicherbereich 6 werden z. B. folgende "administrative Daten" abgespeichert:
● Daten der Anlage (Leisfungs-Einrichtung): kennzeichnende Daten wie Seriennummer, Leistung, Spannung, Aufstellungsort, Eigentümer, Flüssigkeitstyp der Probe (z. B. Öltyp), GPS-Daten des Equipments, bei welchem die ProbeEntnahme erfolgt ist,
● genauer Entnahmeort der Probe am Equipment (wie z. B. "Entnahmehahn Nr. xxx", "Bodenbereich", "oberer Bereich", "mittlerer Bereich"), Entnahmemenge, Entnahmedatum, ausführende Service-Person (Name), Datum und Uhrzeit dieser Probeentnahme, die mittels der Sensorgruppe 20 ermittelten "vorläufigen Probe-Sensorik-Daten",
● anwenderseitige Informationen, wie z. B. Kommenfare über besondere Vorkommnisse (kritische Beobachtungen) während des Entnahmevorganges, die gewünschten, vom Labor zu erstellenden Analysenwerte, z. B. in der Flüssigkeit gelöste Gase, durchzuführende chemische Tests, Furane, usw..

Im zweiten Speicherbereich 7 werden z. B. folgende "administrative Daten" abgespeichert:
● ein während des Entnahmevorganges erzeugter eindeutiger Bestätigungscode mit eindeutiger Zuordnung zur Leistungs-Einrichtung und zum Zeitpunkt dieser Probeentnahme,
● ein Schlüssel für den kontrollierten Zugriff.

Mittels der einzelnen Sensoren 11 - 14 des Sensorschaltkreises 10 können z. B. in der Flüssigkeit enthaltener Wasserstoff, enthaltenes Azetylen, enthaltenes Ethin und enthaltenes Wasser als gewünschte Sensordaten respektive "vorläufige Probe-Sensorik-Daten" ermittelt werden. Mittels der Prozesseinheit 3 und des Signalprozessors 4 können diese Sensordaten weiterverarbeitet und/oder zueinander in Beziehung gesetzt werden und/oder miteinander verbunden werden. Die Resultate werden im ersten Speicherbereich 6 abgespeichert und sind über die Kommunikationsgruppe 18 auslesbar.

Wie bereits vorstehend erwähnt, ist es bevorzugt möglich, die vom Sensor 1 erfassten Sensordaten, d. h. die "administrativen Daten" und die "vorläufigen Probe-Sensorik-Daten" zunächst an ein externes Lesegerät 34 zu übertragen und anschließend von dort an den Behälter-Tag 22 zu übertragen, um dort ein Abspeichern zu ermöglichen. Somit können alle Informationen, wie z. B. Equipment, Probeentnahme-Ort, Service-Person und die Vorort ermittelten Sensordaten respektive "vorläufige Probe-Sensorik-Daten" zusammen mit dem Behälter 24 zum Labor transportiert werden, wobei das Labor die weiteren gewünschten Untersuchungen der entnommenen Probe vornimmt. Der Sensor 1 selbst muss vorteilhaft nicht mit zum Labor transportiert werden, sondern steht (nach erfolgter Reinigung) für die Entnahme weiterer Proben unter Verwendung weiterer Behälter zur Verfügung.

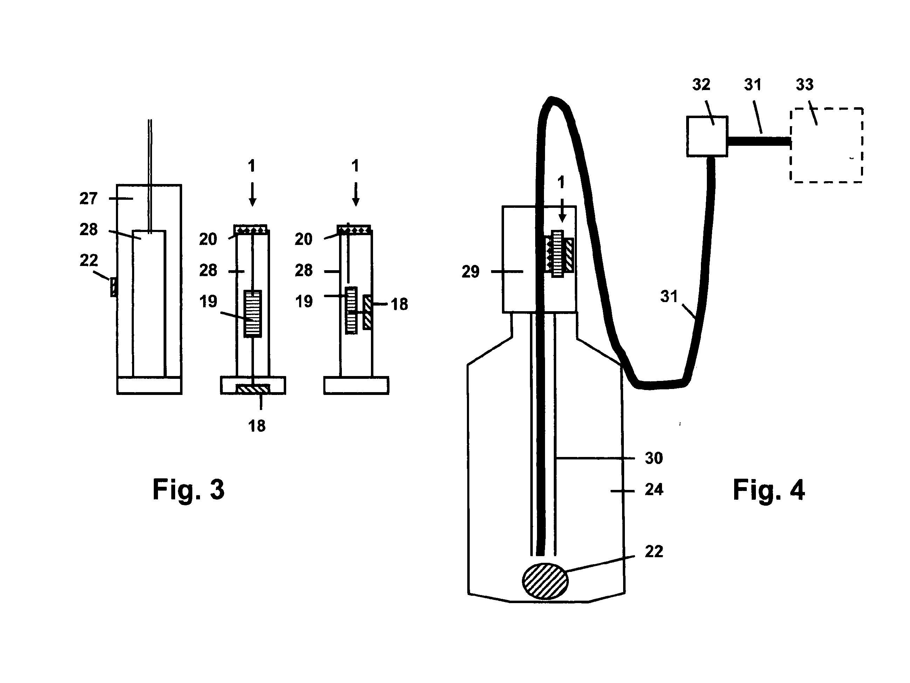

In Fig. 3 ist eine zweite Ausführungsform mit einem Probeentnahme-Behälter für eine Flüssigkeit in Form einer Spritze (quasi eines zweiseitig offenen Behälters) dargestellt, und zwar ist die Spritze im linken Bereich der Figur gezeigt, während im mittleren und im rechten Bereich der Figur zwei unterschiedliche Varianten des zugeordneten Kolbens gezeigt sind. Der Behälter-Tag 22 ist an der Außenwand der Spritze 27 angebracht, z. B. angeklebt. Bei dieser Ausführungsform wird die gewünschte Probe durch Zurückziehen eines Kolbens 28 einer Spritze 27 entnommen. Die Sensoreinheit 1 ist zweckmäßig derart am Kolben 28 angebracht, dass sich die Sensorgruppe 20 an der dem Innenraum der Spritze 27 zugewandten Stirnfläche des Kolbens befindet, um damit rasch in Kontakt mit der entnommenen Probe treten zu können, während die Elektronikgruppe 19 inklusive Energieversorgung geschützt im Innenraum des Kolbens 28 untergebracht ist. Die Kommunikationsgruppe 18 kann entweder an der der Außenatmosphäre zugewandten Stirnfläche des Kolbens 28 (wie im mittleren Bereich der Figur 3 gezeigt) oder an einer Seitenfläche des Kolbens 28 (wie im rechten Bereich der Figur 3 gezeigt) angeordnet sein.

Nach erfolgter Probeentnahme wird der Kolben 28 vollständig von der Spritze 27 abgezogen und beide Stirnflächen der Spritze 27 werden mit geeigneten Kappen verschlossen, worauf der Transport der derart verschlossenen, die Probe enthaltenden Spritze 27 zum Labor erfolgt. Zuvor erfolgt die Datenübertragung zunächst von der Sensoreinheit 1 des Kolbens 28 zum externen Lesegerät 34 und danach vom Lesegerät zum Behälter-Tag 22. Der Kolben 28 inklusive Sensor 1 wird vorzugsweise nicht zum Labor transportiert, sondern steht (nach Reinigung) für weitere Probeentnahmen zur Verfügung.

In Fig. 4 ist eine dritte Ausführungsform mit einem Probeentnahme-Behälter für eine Flüssigkeit mit zugeordneter, lösbarer Durchflusseinheit dargestellt. Der Probeentnahme-Behälter 24 für eine Flüssigkeit ist dabei mit einer Durchflusseinheit 29 verschlossen, welche in Form einer speziellen Kappe ausgeführt ist, die einerseits mit einem Stutzen 30 in den Innenraum des Probeentnahme-Behälter 24 ragt und die andererseits über einen Schlauch 31 und ein Ventil 32 an eine Leistungs-Einrichtung (Anlage) 33, z. B. einen Leistungs-Transformator, angeschlossen ist, aus welcher eine Probe entnommen werden soll. In der Durchflusseinheit 29 ist eine Sensoreinheit 1 eingebaut, deren Sensorgruppe 20 in unmittelbaren Kontakt mit der aus der Leistungs-Einrichtung 33 entnommenen und in den Probeentnahme-Behälter 24 einfließenden Flüssigkeit tritt. Die gewünschten Sensordaten werden während des Einfließens der Flüssigkeit in den Behälter 24 gewonnen. An der Außenwand des Probeentnahme-Behälters 24 ist der Behälter-Tag 22 angebracht, z. B. angeklebt.

Nach erfolgter Probeentnahme wird die Durchflusseinheit 29 vom Probeentnahme-Behälter 24 gelöst, worauf der Probeentnahme-Behälter 24 mittels einer geeigneten Kappe verschlossen wird. Der Transport des derart verschlossenen, die Probe enthaltenden Behälters 24 zum Labor kann erfolgen. Zuvor erfolgt die Datenübertragung zunächst von der Sensoreinheit 1 der Durchflusseinheit 29 zum externen Lesegerät 34 und danach vom Lesegerät zum Behälter-Tag 22. Die Durchflusseinheit 29 inklusive Sensor 1 wird nicht zum Labor transportiert, sondern steht (nach Reinigung) für weitere Probeentnahmen zur Verfügung.

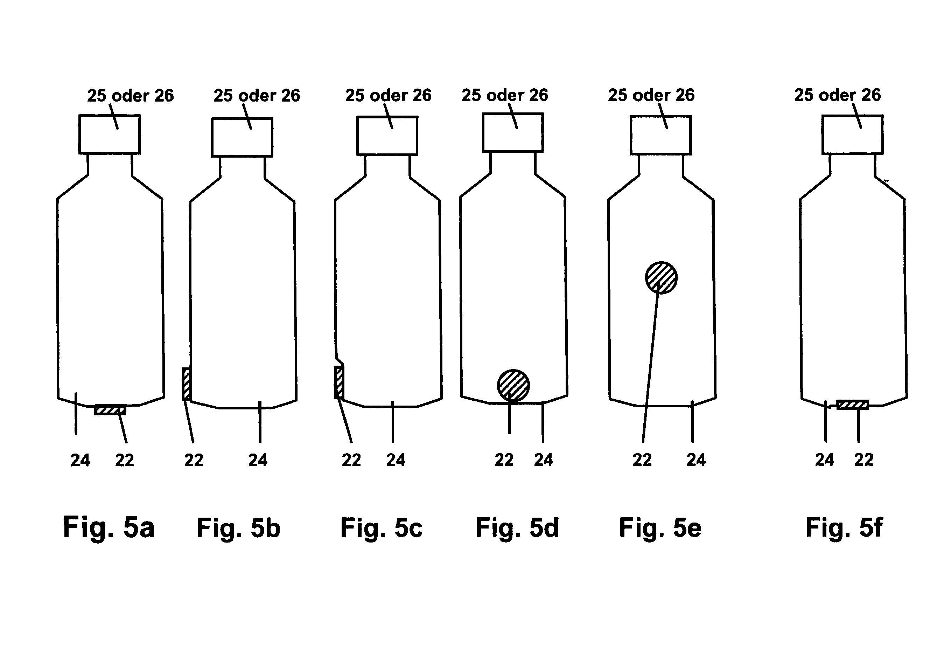

In den Figuren 5a - 5f sind unterschiedliche Ausführungsvarianten von Probeentnahme-Behältern dargestellt:
● Fig. 5a zeigt eine seitliche Ansicht eines Behälters mit einem am Boden des Probeentnahme-Behälters 24 befestigten Behälter-Tag 22.

● Fig. 5b zeigt eine seitliche Ansicht eines Behälters mit einem am Mantel in der Nähe des Bodenbereichs des Probeentnahme-Behälters 24 befestigten Behälter-Tag 22.
● Fig. 5c zeigt eine seitliche Ansicht eines Behälters mit einem an einer zurückspringenden Fläche (Vertiefung) des Mantels in der Nähe des Bodenbereichs des Probeentnahme-Behälters 24 befestigten Behälter-Tag 22.
● Fig. 5d zeigt eine Fronta̅nsicht eines Behälters mit einem am Mantel in der Nähe des Bodenbereichs des Probeentnahme-Behälters 24 befestigten Behälter-Tag 22.
● Fig. 5e zeigt eine Frontansicht eines Behälters mit einem am Mantel im mittleren Bereich des Probeentnahme-Behälters 24 befestigten Behälter-Tag 22.
● Fig. 5f zeigt eine seitliche Ansicht eines Behälters mit einem an einer zurückspringenden Fläche des Bodens des Probeentnahme-Behälters 24 befestigten Behälter-Tag 22, wobei vorzugsweise ein direkter Kontakt zwischen der im Probeentnahme-Behälter 24 befindlichen Flüssigkeit und dem Behälter-Tag 22 besteht.

Ein wichtiges Kriterium bei der Auswahl des Behälter-Bereichs für die Befestigung des Behälter-Tags 22 ist die Forderung, dass die Temperatur der Probe während der Entnahme aus der Leistungs-Einrichtung möglichst genau erfasst werden soll. Bei allen gezeigten Ausführungsvarianten werden die Probeentnahme-Behälter 24 zunächst mittels einer Kappe 26 (für die Ermittlung der gewünschten Sensordaten) und vorzugsweise anschließend mittels einer Kappe 25 (für den Transport des Probeentnahme-Behälters 24 zu einem Labor) verschlossen.

Allgemein gilt für die Sensoreinheit 1, dass sie wahlweise entweder
● in Form einer passiven Einheit ausgeführt sein kann, welche nicht eigenständig sendet, sondern deren Daten von einer aktiven Einheit auszulesen sind, oder
● in Form einer aktiven Einheit ausgeführt sein kann, welche ihre Daten eigenständig aussendet (beispielsweise in fest vorgegebenen zeitlichen Abständen).

Zu Zwecken der Kalibrierung ist es zweckmäßig, die Sensoreinheit 1 in fest vorgegebenen zeitlichen Abständen zum Labor zu transportieren. Dort können die einzelnen Sensoren kalibriert werden. Die bei der Kalibrierung jeweils ermittelten Korrekturwerte werden in der Sensoreinheit abgespeichert und stehen danach für die zukünftige Detektion von "vorläufigen Probe-Sensorik-Daten" zur Verfügung.

### Bezugszeichenliste

- 1: Sensoreinheit
- 2: -
- 3: Prozesseinheit (Mikroprozessor oder Mikrocontroller oder FPGA (Field Programmable Gate Array)
- 4: digitaler Signalprozessor (DSP)
- 5: elektronische Speicherschaltung
- 6: erster Speicherbereich
- 7: zweiter Speicherbereich
- 8: elektronischer RFID-Schaltkreis
- 9: RFID- Antenne
- 10: elektronischer Sensorschaltkreis
- 11: erster Sensor
- 12: zweiter Sensor
- 13: dritter Sensor
- 14: n-ter Sensor
- 15: Batterie / Akkumulator
- 16: induktive Aufladeschaltung
- 17: -
- 18: Kommunikationsgruppe = 8 + 9
- 19: Elektronikgruppe inklusive Energieversorgung = 3 + 4 + 5 + 6 + 7 + 15 + 16
- 20: Sensorgruppe = 10 + 11 + 12 + 13 + 14
- 21: -
- 22: Behälter-Tag, vorzugsweise RFID-Tag oder NFC-Tag
- 23: -
- 24: Probeentnahme-Behälter für eine Flüssigkeit
- 25: Kappe
- 26: Kappe inklusive Sensoreinheit
- 27: Spritze
- 28: Kolben
- 29: Durchflusseinheit
- 30: Stutzen
- 31: Schlauch
- 32: Ventil
- 33: Leistungs-Einrichtung (Anlage)
- 34: externes Lesegerät

## Patentansprüche

1. Probeentnahme-Behälter (24, 27) für eine Flüssigkeit mit Behälter-Tag (22) und zugeordneter, lösbarer Sensoreinheit (1),
● wobei der Behälter-Tag (22) fest am Probeentnahme-Behälter (24, 27) angebracht ist und einen via Funk auslesbaren Lese-Speicherbereich aufweist, welcher eine eindeutige Identifikation des Probeentnahme-Behälters (24, 27) ermöglicht,
● wobei die zugeordnete, lösbare Sensoreinheit (1) während der Probeentnahme lösbar am Probeentnahme-Behälter (24, 27) befestigt ist und eine Elektronikgruppe (19) inklusive Prozesseinheit (3), elektronischer Speicherschaltung (5) und Energieversorgung (15), eine via Funk zu betreibende Kommunikationsgruppe (18) sowie eine Sensorgruppe (20) aufweist, welche Vorort während der Entnahme der Flüssigkeit aus einer Leistungs-Einrichtung (33) "vorläufige Probe-Sensorik-Daten" ermittelt,
● wobei der Behälter-Tag (22) des Weiteren einen via Funk beschreibbaren/auslesbaren Schreib/Lese-Speicherbereich aufweist, welcher ein Abspeichern der von der lösbaren Sensoreinheit (1) erfassten "vorläufigen Probe-Sensorik-Daten" sowie der diese Probeentnahme betreffenden "administrativen Daten" ermöglicht.

2. Probeentnahme-Behälter (24) nach Anspruch 1, **dadurch gekennzeichnet, dass** die Sensoreinheit (1) in einer den Probeentnahme-Behälter (24) verschließenden Kappe (26) derart installiert ist, dass die entnommene Probe die Sensorgruppe (20) kontaktiert.

3. Probeentnahme-Behälter (27) nach Anspruch 1, **dadurch gekennzeichnet, dass** der Probeentnahme-Behälter in Form einer Spritze (27) ausgebildet ist und die Sensoreinheit (1) im Kolben (28) dieser Spritze derart installiert ist, dass die entnommene Probe die Sensorgruppe (20) kontaktiert.

4. Probeentnahme-Behälter (24) nach Anspruch 1, **dadurch gekennzeichnet, dass** die Sensoreinheit (1) in einer den Probeentnahme-Behälter (24) verschließenden Durchflusseinheit (26) derart installiert ist, dass die entnommene Probe die Sensorgruppe (20) kontaktiert.

5. Probeentnahme-Behälter (24) nach einem der vorstehenden Ansprüche, **dadurch gekennzeichnet, dass** der Behälter-Tag (22) als RFID-Tag ausgebildet ist.

6. Probeentnahme-Behälter (24) nach Anspruch 5, **dadurch gekennzeichnet, dass** der Behälter-Tag (22) bei Ausbildung in Form eines passiven RFID-Tags einen Temperatursensor zur Detektion der Augenblickstemperatur der Flüssigkeit während der Probeentnahme aufweist.

7. Probeentnahme-Behälter (24) nach Anspruch 5, **dadurch gekennzeichnet, dass** der Behälter-Tag bei Ausbildung in Form eines aktiven RFID-Tags einen Temperatursensor zur Detektion der während der Probeentnahme auftretenden Temperaturkurve aufweist, aus welcher eine typische Temperatur ableitbar ist.

8. Probeentnahme-Behälter (24) nach einem der Ansprüche 1 - 4, **dadurch gekennzeichnet, dass** der Behälter-Tag (22) als NFC-Tag ausgebildet ist.

9. Probeentnahme-Behälter (24) nach einem der vorstehenden Ansprüche, **dadurch gekennzeichnet, dass** die Kommunikationsgruppe (18) der Sensoreinheit (1) als RFID-Schaltkreis ausgebildet ist.

10. Probeentnahme-Behälter (24) nach einem der Ansprüche 1 - 8, **dadurch gekennzeichnet, dass** die Kommunikationsgruppe (18) der Sensoreinheit (1) als NFC-Schaltkreis ausgebildet ist.

11. Probeentnahme-Behälter (24) nach einem der vorstehenden Ansprüche, **dadurch gekennzeichnet, dass** die Sensorgruppe (10) einen Sensor zur Ermittlung von gelöstem Wasserstoff und/oder einen Sensor zur Ermittlung von enthaltenem Azetylen und/oder einen Sensor zur Ermittlung von enthaltenem Ethin und/oder einen Sensor zur Ermittlung von enthaltenem Wasser und/oder einen Temperatursensor aufweist.
